(19) Europäisches Patentamt
European Patent Office
Office européen des brevets

(11) Veröffentlichungsnummer: **0 243 940**
**B1**

# (12) EUROPÄISCHE PATENTSCHRIFT

(45) Veröffentlichungstag der Patentschrift: **18.04.90**

(51) Int. Cl.⁴: **C07D 207/06**, C07D 207/08, C07D 209/48, C07D 207/12, C07D 209/52, A01N 43/36, A01N 43/38 // C07D207/267, C07D207/408

(21) Anmeldenummer: **87106164.4**

(22) Anmeldetag: **28.04.87**

## (54) Phenyl-und Cyclohexyl-Isobutylpyrrolidinderivate.

(30) Priorität: **02.05.86 DE 3614906**

(43) Veröffentlichungstag der Anmeldung: **04.11.87 Patentblatt 87/45**

(45) Bekanntmachung des Hinweises auf die Patenterteilung: **18.04.90 Patentblatt 90/16**

(84) Benannte Vertragsstaaten: **AT BE CH DE ES FR GB GR IT LI NL SE**

(56) Entgegenhaltungen:
**EP-A- 0 000 333**
**DE-A- 2 830 127**

**PESTIC. SCI. Band 15, 1984, Seiten 285-295; E.H. POMMER "Chemical Structure-fungicidal activity relationships in substituted morpholines"**
**ANGEWANDTE CHEMIE, Band 19, 1980, Seiten 184-189; W. HIMMELE et al.: "3-Phenylpropylamines, A new class of systemic fungicides"**
**CHEMICAL ABSTRACTS, Band 92, 1980, Seite 167, Spalte 1, Zusammenfassungsnr. 192461v, Columbus, Ohio, US; E.H. POMMER et al.: "Control of cereal diseases with fenpropemorph, a new morpholine derivative"**

(73) Patentinhaber: **BASF Aktiengesellschaft, Carl-Bosch-Strasse 38, D-6700 Ludwigshafen(DE)**

(72) Erfinder: **Buschmann, Ernst, Dr., Georg-Ludwig-Krebs-Strasse 10, D-6700 Ludwigshafen(DE)**
Erfinder: **Himmele, Walter, Dr., Eichenweg 14, D-6909 Walldorf(DE)**
Erfinder: **Siegel, Hardo, Dr., Hans-Purrmann-Allee 25, D-6720 Speyer(DE)**
Erfinder: **Eckhardt, Heinz, Dr., Ruedigerstrasse 7, D-6700 Ludwigshafen(DE)**
Erfinder: **Ammermann, Eberhard, Dr., Sachsenstrasse 3, D-6700 Ludwigshafen(DE)**
Erfinder: **Pommer, Ernst-Heinrich, Dr., Berliner Platz 7, D-6703 Limburgerhof(DE)**

**EP 0 243 940 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Phenyl-und Cyclohexyl-isobutylpyrrolidine der allgemeinen Formel I

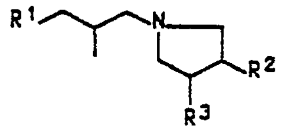

(I)

in der die Substituenten folgende Bedeutung haben:
$R^1$ 4-tert.-Butylphenyl oder 4-tert.-Butylcyclohexyl,
$R^2$ $C_5$–$C_8$-Alkyl, $C_2$–$C_4$-Alkenyl, $C_1$–$C_4$-Alkoxy, $C_2$–$C_4$-Alkenyloxy, $C_1$–$C_4$-Hydroxyalkyl, $C_2$–$C_4$-Alkoxyalkyl, $C_3$–$C_8$-Alkenyloxyalkyl, $C_2$–$C_4$-Acyloxyalkyl, $C_3$–$C_8$-Alkylcarbonylakyl, und für den Fall, daß $R^1$ 4-tert.-Butylphenyl bedeutet, zusätzlich die Methylgruppe, und für den Fall, daß $R^1$ 4-tert.-Butylcyclohexyl bedeutet, zusätzlich $C_1$–$C_4$-Alkyl, die Phenyl-oder Hydroxylgruppe,
$R^3$ Wasserstoff, wobei
$R^2$ und $R^3$ auch gemeinsam einen carbocyclischen Rest bedeuten können,
sowie deren pflanzenphysiologisch verträgliche Salze.

Außerdem betrifft die Erfindung die Herstellung der Verbindungen I, ihre Verwendung als Fungizide, sowie Fungizide, die die Verbindungen I enthalten.

Aus der DE-OS 2 727 482 ist das Phenylpyrrolidinderivat I' und dessen Hydrochlorid bekannt.

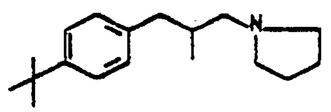

(I')

Es ist ferner bekannt, N-Arylpropyl-substituierte Amine oder N-Arylpropyl-substituierte Morpholine als Fungizide zu verwenden (DE-A 2 830 172, Pestic. Sci. 15 (1984), S. 285–295).

Der Erfindung lag die Aufgabe zugrunde, neue Verbindungen dieses Typs mit verbesserten Eigenschaften als Fungizide bereitzustellen.

Demgemäß wurden die eingangs definierten neuen Phenyl-und Cyclohexyl-isobutylpyrrolidine I gefunden. Weiterhin wurde gefunden, daß sich die Verbindungen I hervorragend als Fungizide eignen.

Außerdem wurden Verfahren zur Herstellung der Verbindungen I gefunden.

Die Verbindungen I sind im einzelnen nach folgenden Methoden erhältlich:

a) Umsetzung einer Verbindung II mit einem Pyrrolidinderivat III

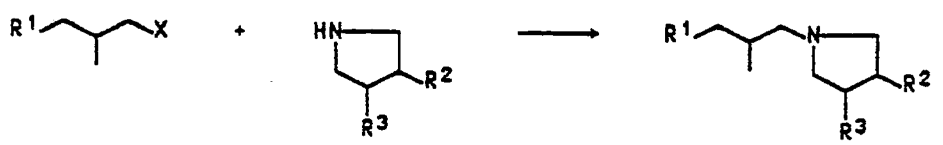

(II) (III) (I)

unter basischen Bedingungen. Als Reste X seien beispielsweise genannt Chlor, Brom, Jod, die Methandie Benzol-oder die Toluolsulfonylgruppe.

Die Reaktion wird bei 100 bis 200°C gegebenenfalls in Gegenwart eines inerten Lösungsmittels durchgeführt. Als Basen werden anorganische Basen bevorzugt, beispielsweise Kalium-, Natrium- und Lithiumhydroxid, Natriumhydrid und Natrium- und Kaliumcarbonat. Geeignet sind auch organische Basen wie z.B. Triethylamin, Dicyclohexylamin und Diisopropylamin. Die Reaktion läßt sich auch mit einem Überschuß des Pyrrolidinderivates III, das auch als Reaktionspartner fungiert, durchführen. Um die entsprechenden Reaktionstemperaturen zu erreichen, empfiehlt sich u.U. das Arbeiten unter Druck (etwa bis zu 10 bar).

b) Umsetzung eines Aldehydes IV mit einem Pyrrolidinderivat III unter gleichzeitiger oder anschließender Hydrierung.

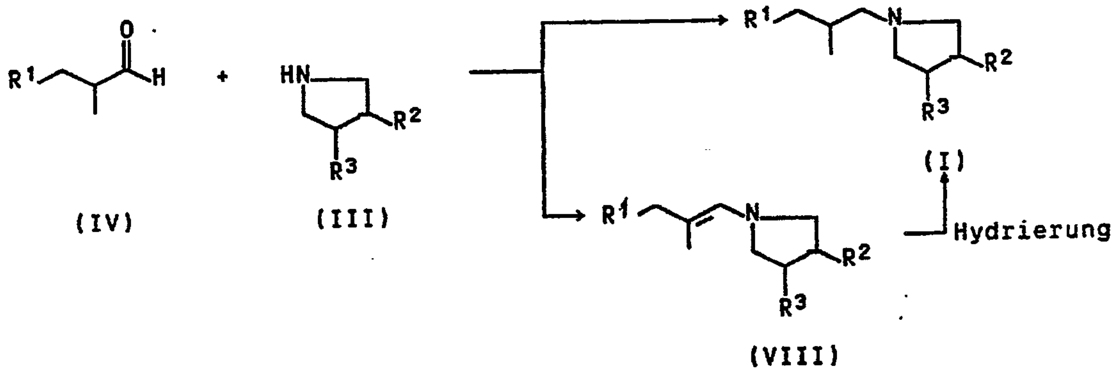


(IV) (III) (I) (VIII)

$b_1$) Bei der direkten Methode zur Herstellung der Verbindungen I wird ein Gemisch aus III und IV in Gegenwart eines Lösungsmittels, beispielsweise Methanol, Ethanol, Tetrahydrofuran oder Toluol, und eines Hydrierkatalysators wie z.B. Raney-Nickel, Platin-IV-oxid, $Ru_2O_3$ oder Palladium auf Kohle im Autoklaven auf 100 bis 150°C erwärmt. Nach einer Vorlaufzeit von 30 Minuten wird Wasserstoff bis zu Druckkonstanz aufgepreßt.

$b_2$) Bei der Zweistufen-Reaktion wird das Enamin VIII in an sich bekannter Weise unter wasserentziehenden Bedingungen hergestellt und anschließend mit einem Edelmetallkatalysator wie Raney-Nickel, Raney-Cobalt, $PtO_2$ oder $Ru_2O_3$, vorzugsweise Palladium auf Kohle mit Wasserstoff reduziert.

c) Umsetzung eines Aminderivats V mit einer Verbindung VIa, VIb oder VIc

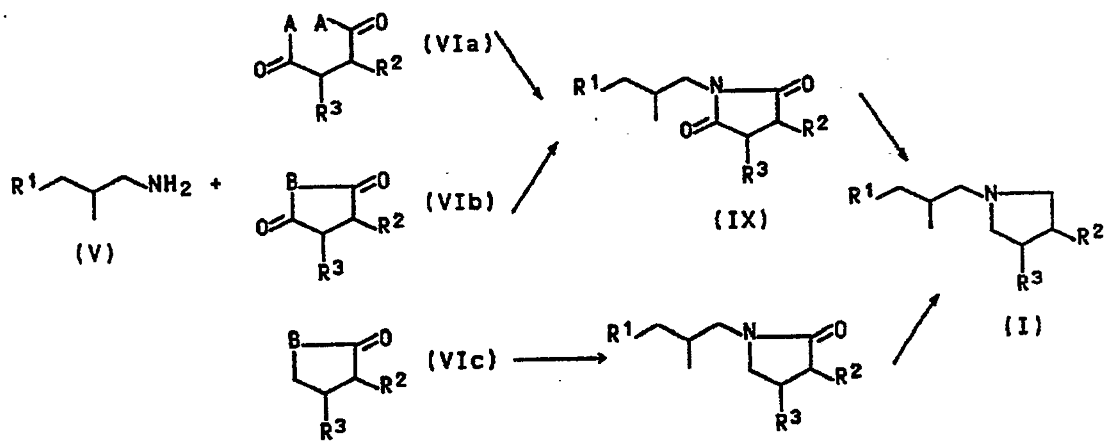

(VIa) (VIb) (VIc) (V) (IX) (X) (I)

B = Sauerstoff; NH

und anschließender Reduktion der Carbonylgruppe(n). Bei der Umsetzung von Dicarbonsäuren, Bernsteinsäureanhydriden und 5-Ringlactonen wird das Reaktionswasser entweder am Wasserabscheider mit einem geeigneten Schleppmittel oder in Gegenwart eines wasserbindenden Mittels aus dem Gleichgewicht entfernt. Als Lösungsmittel bei der Wasserabscheidung eignen sich höher siedende Kohlenwasserstoffe wie Toluol, Xylol, Chlorbenzol oder Ligroin.

Als wasserbindende Verbindungen eignen sich Acetanhydrid, Hexachlorethyltriphenylphosphin oder Molekularsiebe.

Als Lösungsmittel bei dieser Reaktionsführung eignen sich Kohlenwasserstoffe wie Toluol oder Xylol. Halogenkohlenwasserstoffe wie Chlorbenzol, Ether wie Tetrahydrofuran oder Dioxan, in einzelnen Fällen auch Alkohole wie Methanol, Ethanol oder Isopropanol.

Es kann hierbei jedoch auch ohne Lösungsmittel gearbeitet werden. Die Reaktionen lassen sich bei Temperaturen ab 100°C durchführen. Falls die Reaktionstemperatur unter Normaldruck nicht erreichbar ist, wird im Autoklaven unter dem Eigendruck der Reaktionsmischung bei der benötigten Umsetzungstemperatur gearbeitet.

Die Dicarbonsäuredihalogenide lassen sich bei -20 bis +100°C, vorzugsweise bei 0 bis +10°C in Gegenwart einer Base, die zusätzlich als Lösungsmittel dienen kann, wie beispielsweise Diisopropylamin, Tributylamin, Pyridin, Picolin, Triethylamin, Dicyclohexylethylamin oder einer Base wie beispielsweise Natriumcarbonat, Kaliumcarbonat oder Natriumhydrogencarbonat in Gegenwart eines inerten Lösungsmittels wie Chloroform, Dichlormethan oder Tetrahydrofuran umsetzen.

Die Umsetzung der Aminderivate V mit einem 5-Ringlactamderivat VIII findet bei hohen Temperaturen, die zwischen 150 und 200°C liegen, leichter statt, wobei die benötigte Reaktionstemperatur gegebenenfalls nur in einem Autoklaven erreicht werden kann. Die Reaktion kann in einem inerten Lösungsmittel wie beispielsweise Toluol, Xylol, Ethanol, Isopropanol oder Cyclohexanol erfolgen oder auch in Abwesenheit eines Lösungsmittels.

Zur Reduktion der Carbonylverbindungen zu Alkylverbindungen kann man mit einem Reduktionsmittel bei -20 bis +100°C, vorzugsweise 0 bis +10°C umsetzen. Als Reduktionsmittel sind Hydride wie Lithiumaluminiumhydrid und Diboran bevorzugt.

Die Reaktion kann auch unter Wolff-Kishner-Reaktionsbedingungen oder elektrochemisch erfolgen.

Geeignete Lösungsmittel für die Hydridreduktion sind Ether wie Diethylether, Diisopropylether, Tetrahydrofuran und Dioxan, Kohlenwasserstoffe wie Xylol und Toluol.

d) Verbindungen Ia

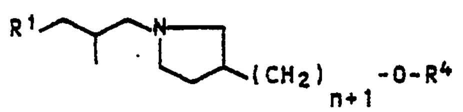

(Ia)

$R^4$= H, $C_1$–$C_4$-Alkyl, $C_3$–$C_8$-Alkylcarbonyl, $C_2$–$C_4$-Alkenyl, $C_2$–$C_4$-Acyl,
n = 0 bis 3
sind über die Zwischenstufe XI zugänglich. Die Umsetzung eines 2,5-Dimethoxy-tetrahydrofuranderivates VII mit einem Aminderivate V in Gegenwart einer Carbonsäure, die zugleich als Lösungsmittel wie Toluol oder Xylol führt bei Temperaturen von 50 bis 200°C, vorzugsweise 80 bis 150°C, zu den Zwischenprodukten XI.

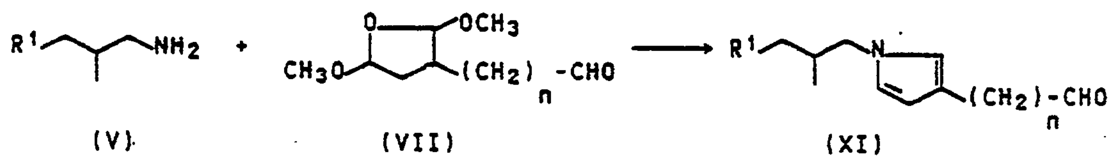

(V) (VII) (XI)

Als Carbonsäuren eignen sich beispielsweise Ameisensäure, Essigsäure und Propionsäure.

Das entstandene Pyrrolaldehydderivat XI läßt sich in einem inerten Lösungsmittel mit Wasserstoff in Gegenwart eines Edelmetallkatalysators reduzieren.

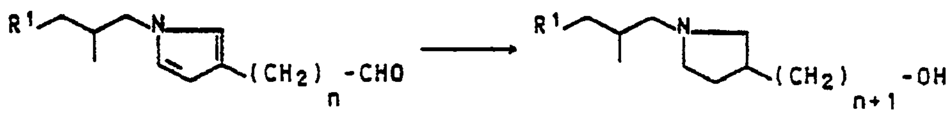

Als inerte Lösungsmittel eignen sich Tetrahydrofuran und Dioxan.

Als Edelmetallkatalysatoren werden $PtO_2$, $Ru_2O_3$ und Palladium auf Kohle bevorzugt.

Gegebenenfalls lassen sich die gebildeten Alkohole mit einer Verbindung $R^4$–X, in der X eine Abgangsgruppe wie Chlor, Brom, Jod, die Methan-, Benzol-oder Toluolsulfongruppe nach üblichen Methoden verethern oder verestern, wodurch eine explizite Beschreibung dieser Methoden entbehrlich ist.

Zur Synthese der Verbindungen I kann man von tert.-Butylphenylverbindungen ausgehen. Die Hydrierung des Aromaten zur Herstellung der tert.-Butylcyclohexylverbindungen kann zu Anfang der Reaktionssequenz oder auf einer beliebigen Stufe, besonders aber auch als letzte Reaktion ausgeführt werden. Die Hydrierung läßt sich mit Wasserstoff und katalytischen Mengen eines geeigneten Edelmetallkatalysators gegebenenfalls unter Druck und höheren Temperaturen durchführen. Als Katalysatoren werden $PtO_2$ und $Ru_2O_3$ bevorzugt.

Als Lösungsmittel eignen sich Tetrahydrofuran und Dioxan.

Als Druckbereich werden 100 bis 400 bar bevorzugt, besonders geeignet ist der Bereich zwischen 250 und 350 bar. Als Temperaturbereich wird 100 bis 150°C bevorzugt.

Unter den Resten $R^1$ wird 4-tert.-Butylcyclohexyl bevorzugt und 4-tert.-Butylphenyl besonders bevorzugt.

Der Rest $R^2$ steht für folgende Substituenten:

– $C_5$–$C_8$-Alkyl, wie n-Pentyl, iso-Pentyl, neo-Pentyl, n-Hexyl, iso-Hexyl und n-Octyl,

– für den Fall, daß $R^1$ 4-tert.-Butylphenyl bedeutet, besonders bevorzugt Methyl,
– für den Fall, daß $R^1$ 4-tert.-Butylcyclohexyl bedeutet, $C_1$–$C_4$-Alkyl, wie Methyl, Ethyl, n-Propyl, iso-Propyl, n-Butyl, iso-Butyl und tert.-Butyl,
– für den Fall, daß $R^1$ 4-tert.-Butylcyclohexyl bedeutet, Hydroxy,
– für den Fall, daß $R^1$ 4-tert.-Butylcyclohexyl bedeutet, Phenyl,
– $C_2$–$C_4$-Alkenyl, wie Vinyl, Allyl und Crotyl,
– $C_1$–$C_4$-Alkoxy, wie Methoxy, Ethoxy, n-Propoxy, iso-Propoxy, n-Butoxy, iso-Butoxy und tert.-Butoxy,
– $C_2$–$C_4$-Alkenyloxy, wie Vinyloxy, Allyloxy und Crotyloxy,
– $C_1$–$C_4$-Hydroxyalkyl, wie Hydroxymethyl und Hydroxyethyl,
– $C_2$–$C_4$-Alkoxyalkyl, wie Methoxymethyl, Methoxyethyl, Ethoxymethyl, Ethoxyethyl und Methoxy-n-propyl,
– $C_3$–$C_8$-Alkenyloxyalkyl, wie Vinyloxymethyl, Vinyloxyethyl,Allyloxymethyl, Allyloxyethyl, Crotyloxymethyl und Crotyloxyethyl,
–$C_2$–$C_4$-Acyloxyalkyl, wie Formyloxymethyl, Formyloxyethyl, Acetoxymethyl, Acetoxyethyl, Acetoxy-n-propyl und Propionyloxymethyl,
$C_3$–$C_8$-Alkylcarbonylalkyl, wie Methylcarbonylmethyl, Methylcarbonylethyl, Ethylcarbonylmethyl und Ethylcarbonylmethyl.

Unter den Resten $R^3$ wird Wasserstoff besonders bevorzugt.

$R^2$ und $R^3$ können zu einem carbocyclischen Ring verknüpft sein, beispielsweise in den folgenden bicyclischen Pyrrolidinen:

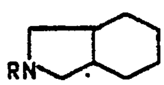
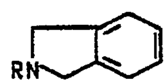
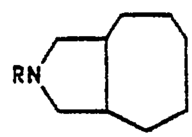

Besonders bevorzugt wird

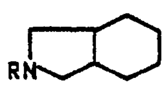 , 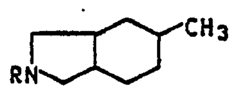 und 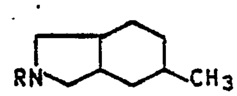

Pflanzenverträgliche Salze der Amine der Formel I können beispielsweise gebildet werden mit: HCl, HBr, HJ, $H_2SO_4$, $H_3PO_4$, $HCO_2H$, $CH_3CO_2H$, $CH_3CH_2CO_2H$, $C_{12}H_{25}$–$C_6H_5$–$SO_3H$. Besonders bevorzugt wird HCl.

Beispiele für geeignete Verbindungen I sind:

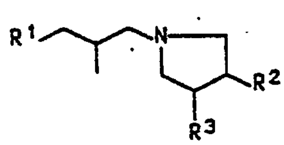

(I)

| $R^1$ | $R^2$ | $R^3$ |
|---|---|---|
| 4-tert.-Butylphenyl | $-CH_3$ | H bzw. dessen Hydrochlorid |
| 4-tert.-Butylphenyl | $-CH_2CH(CH_3)CH_2CH_3$ | H bzw. dessen Hydrochlorid |
| 4-tert.-Butylphenyl | -n-$C_6H_{13}$ | H bzw. dessen Hydrochlorid |
| 4-tert.-Butylphenyl | $-CH_2CH_2OH$ | H bzw. dessen Hydrochlorid |
| 4-tert.-Butylphenyl | $-CH_2CH_2-O-CH_3$ | H bzw. dessen Hydrochlorid |
| 4-tert.-Butylphenyl | $-CH_2CH_2-O-CH_2-CH_3$ | H bzw. dessen Hydrochlorid |
| 4-tert.-Butylphenyl | $-CH_2CH_2-O-CO-CH_3$ | H bzw. dessen Hydrochlorid |
| 4-tert.-Butylphenyl | $-CH_2CH_2-O-CH_2-CH=CH_2$ | H bzw. dessen Hydrochlorid |
| 4-tert.-Butylphenyl | $-(CH_2)_4-$ | bzw. dessen Hydrochlorid |
| 4-tert.-Butylcyclohexyl | $-CH_3$ | H bzw. dessen Hydrochlorid |
| 4-tert.-Butylcyclohexyl | $-C_2H_5$ | H bzw. dessen Hydrochlorid |
| 4-tert.-Butylcyclohexyl | -n-$C_3H_7$ | H bzw. dessen Hydrochlorid |
| 4-tert.-Butylcyclohexyl | -iso-$C_3H_7$ | H bzw. dessen Hydrochlorid |
| 4-tert.-Butylcyclohexyl | -n-$C_4H_9$ | H bzw. dessen Hydrochlorid |
| 4-tert.-Butylcyclohexyl | -iso-$C_4H_9$ | H bzw. dessen Hydrochlorid |
| 4-tert.-Butylcyclohexyl | -tert.-$C_4H_9$ | H bzw. dessen Hydrochlorid |
| 4-tert.-Butylcyclohexyl | -n-$C_6H_{13}$ | H bzw. dessen Hydrochlorid |
| 4-tert.-Butylcyclohexyl | $-CH_2-OH$ | H bzw. dessen Hydrochlorid |
| 4-tert.-Butylcyclohexyl | $-CH_2-O-CH_3$ | H bzw. dessen Hydrochlorid |
| 4-tert.-Butylcyclohexyl | $-CH_2-O-CH_2CH=CH_2$ | H bzw. dessen Hydrochlorid |
| 4-tert.-Butylcyclohexyl | $-CH_2-O-CO-CH_3$ | H bzw. dessen Hydrochlorid |
| 4-tert.-Butylcyclohexyl | $-(CH_2)_4-$ | bzw. dessen Hydrochlorid |
| 4-tert.-Butylcyclohexyl | $-CH_2CH(CH_3)CH_2CH_2-$ | bzw. dessen Hydrochlorid |

Die neuen Pyrrolidinderivate der Formel I enthalten mindestens ein, zumeist aber mehrere chirale Zentren. Sie werden im allgemeinen als Stereoisomerengemische erhalten. Für die Anwendung der Verbindungen I als Fungizide sind sowohl die diastereomerenreinen bzw. enantiomerenreinen Verbindungen I als auch die bei der Synthese anfallenden Stereoisomerengemische geeignet.

Die Phenyl- und Cyclohexyl-isobutylpyrrolidinderivate I und deren pflanzenphysiologisch verträglichen Salze können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Toluol, Benzol), chlorierte Aromaten (z.B. Chlorbenzole), Paraffine (z.B. Erdölfraktionen), Alkohole (z.B. Methanol, Butanol), Ketone (z.B. Aceton, Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate), Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitblaugen und Methylcellulose.

Die Verbindungen I und deren Salze werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Beispielsweise können die Phenyl- und Cyclohexyl-isobutylpyrrolidinderivate der Formel I und deren Salze mit den folgenden Fungiziden kombiniert werden:

Schwefel,
Dithiocarbamate und deren Derivate, wie
Ferridimethyl-, Zinkdimethyl-, Zinkethylenbis-, Manganethylenbis-, Mangan-Zink-ethylendiamin-bis- oder Zink-(N,N'-propylen-bis-dithiocarbamat),

Ammoniakkomplexe von Zink-(N,N'-ethylen-bis-dithiocarbamat) und von Zink-(N,N'-propylen-bis-dithiocarbamat),
Tetramethylthiuramdisulfide oder
N,N'-Polypropylen-bis-(thiocarbamoyl)-disulfid,

Nitroverbindungen, wie
Dinitro-(1-methylheptyl)-phenylcrotonat,
2-sec.-Butyl-4,6-dinitrophenyl-3,3-dimethylacrylat,
2-sec.-Butyl-4,6-dinitrophenyl-isopropylcarbonat oder
5-Nitro-isophthalsäure-diisopropylester,

Heterocyclen, wie
N-Cyclohexyl-N-methoxy-2,5-dimethyl-furan-3-carbonsäureamid,
2,5-Dimethyl-furan-3-carbonsäurecyclohexylamid,
2-Methyl-furan-3-carbonsäureanilid,
2,5-Dimethyl-furan-3-carbonsäureanilid,
2,4,5-Trimethyl-furan-3-carbonsäureanilid,
DL-Methyl-N-(2,6-dimethyl-phenyl)-N-furoyl(2)-alaninat,
0,0-Diethylphthalimidophosphonothioat,
N-Trichlormethylthio-phthalimid,
N-(1,1,2,2-Tetrachlorethylthio)-tetrahydrophthalimid,
N-Trichlormethylthio-tetrahydrophthalimid,
2-Heptadecyl-2-imidazolin-acetat,
2-(Furyl)-(2)-benzimidazol,
2-Methoxycarbonylamino-benzimidazol,
1-(Butylcarbamoyl)-2-benzimidazol-carbaminsäuremethylester,
2-(Thiazolyl)-(4))-benzimidazol,
N-(n-Propyl)-N-(2,4,6-trichlorphenoxyethyl)-N'-imidazol-yl-harnstoff,
5-Methyl-5-vinyl-3-(3,5-dichlorphenyl)-2,4-dioxo-1,3-oxazolidin,
3-(3,5-Dichlorphenyl)-5-methyl-5-methoxymethyl-1,3-oxazolidin-2,4-dion,
4-(2-Chlorphenylhydrazono)-3-methyl-5-isoxazolan,
2-Thio-1,3-dithio-(4,5-b)-chinoxalin,
2,3-Dihydro-5-carboxanilido-6-methyl-1,4-oxathiin,
2,3-Dihydro-5-carboxanilido-6-methyl-1,4-oxathiin-4,4-dioxid,
2-Rhodamethylthiobenzthiazol,
1-[2-(2,4-Dichlorphenyl)-4-ethyl-1,3-dioxolan-2-yl-ethyl]-1H-1,2,4,-triazol,
1-[2-(2,4-Dichlorphenyl)-4-n-propyl-1,3-dioxolan-2-yl-ethyl]-1H-1,2,4-triazol,
1-(2-(2,4-Dichlorphenyl)-pentyl)-1H-1,2,4-triazol,
5-Amino-1-[bis-(dimethylamino)-phosphinyl]-3-phenyl-1,2,4-triazol,
1-[bis-(4-Fluorphenyl)-methylsilyl]-methyl-1H-1,2,4-triazol,
1-(4-Chlorphenoxy)-3,3-dimethyl-1-(1H-1,2,4-triazol-1-yl)-2-butanon,
1-(4-Phenylphenoxy)-3,3-dimethyl-1-(1H-1,2,4-triazol-1-yl)-2-butanol,
2,4'-Difluor-α-(1H-1,2,4-triazolyl-1-methyl)-benzhydrylalkohol,
1-(4-Phenylphenoxy)-3,3-dimethyl-1-(1H-1,2,4-triazol-1-yl)-2-butanol,
5-Ethoxy-3-trichlormethyl-1,2,3-thiadiazol,
2-Methyl-5,6-dihydro-4-H-pyran-3-carbonsäure-anilid,
N-[3-p-tert.-Butylphenyl)-2-methylpropyl]-piperidin,
3-[3-(3,5-Dimethyl-2-oxycyclohexyl)-2-hydroxyethyl]-glutarimid,
2,6-Dimethyl-N-cyclododecyl-morpholin bzw. 2,6-Dimethyl-N-tridecylmorpholin und deren Salze,
N-Formyl-N-morpholin-2,2,2-trichlorethylacetal,
N-[3-(p-tert.-Butylphenyl)-2-methylpropyl]-cis-2,6-dimethylmorpholin,
Piperazin-1,4-diyl-bis-[1-(2,2,2-trichlor-ethyl)-formamid,
Pyridin-2-thio-1-oxid, Bis-(p-chlorphenyl)-3-pyridinmethanol,
N-(3-Chlor-2,6-dinitro-4-trifluormethylphenyl)-5-trifluormethyl-3-chlor-2-aminopyridin,
8-Hydroxychinolin bzw. dessen Kupfersalz,
5-Butyl-2-dimethylamino-4-hydroxy-6-methyl-pyrimidin,
α-(2-Chlorphenyl)-α-(4-chlorphenyl)-5-pyrimidinmethanol,
2,4-Dichlor-6-(o-chloranilino)-s-triazin oder
N-(2,6-Dimethylphenyl)-N-chloracetyl-D,L-2-aminobutyrolacton,

sowie andere Fungizide, wie
1-(3,4-Dichloranilino)-1-formylamino-2,2,2-trichlorethan,
Dodecylguanidinacetat,
3-(3,5-Dichlorphenyl)-1-isopropylcarbamoylhydantoin,
Hexachlorbenzol,

1,4-Dichlor-2,5-dimethoxybenzol,
1,2-Bis-(3-methoxycarbonyl-2-thioureido)-benzol,
1,2-Bis-(3-ethoxycarbonyl-2-thioureido)-benzol,
2-Methyl-benzoesäure-anilid,
2-Iod-benzoesäure-anilid,
2,3-Dicyano-1,4-dithioanthrachinon,
DL-N-(2,6-Dimethylphenyl)-N-(phenylacetyl)-alaninmethylester,
2-Cyano-N-[(ethylaminocarbonyl)-2-methoximino]-acetamid,
N-(3,5-Dichlorphenyl)-1,2-dimethylcyclopropan-1,2-dicarbonsäureimid,
N-Dichlorfluormethylthio-N',N'-dimethyl-N-phenyl-schwefelsäurediamid oder
DL-N-(2,6-Dimethyl-phenyl)-N-(2'-methoxyacetyl)-alaninmethylester.

Die Phenyl- und Cyclohexyl-isobutylpyrrolidinderivate der Formel I und deren pflanzenverträgliche Salze zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais,k Rasen, Baumwolle, Soja, Kaffee, Zuckerrohr, Obst und Zierpflanzen im Gartenbau, Weinbau sowie Gemüse - wie Gurken, Bohnen und Kürbisgewächse.

Die Verbindungen I eignen sich besonders zur Bekämpfung der aufgeführten Pflanzenkrankheiten in/an den entsprechenden Nutzpflanzen.

| Pflanzenkrankheiten | Nutzpflanzen |
|---|---|
| Erysiphe graminis, Puccinia-Arten | Getreide |
| Septoria nodorum | Weizen |
| Helminthosporium teres | Gerste |
| Pseudocercosporella herpotrichoides | Weizen, Gerste |
| Ustilago-Arten | Getreide, Zuckerrohr |
| Pyricularia oryzae | Reis |
| Cercospora arachidicola | Erdnüsse |
| Podosphaera leucotricha, Venturia inaequalis | Äpfel |
| Botrytis cinerea | Erdbeeren, Reben |
| Uncinula necator | Reben |
| Rhizoctonia-Arten | Baumwolle |
| Erysiphe cichoracearum, Sphaerotheca fuliginea | Kürbisgewächse |
| Fusarium-, Verticillium-Arten | verschiedene Pflanzen |

Außerdem eignen sich einige der Verbindungen auch zur Bekämpfung hautpathogener Pilze wie z.B. Trichophyton mentagrophytes und Candida albicans.

Die fungiziden Mittel enthalten im allgemeinen zwischen 0,1 und 95 Gew.%, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je Hektar. Die neuen Verbindungen können auch im Materialschutz eingesetzt werden. Bei Anwendung der Wirkstoffe im Materialschutz u.a. zur Bekämpfung holzzerstörender Pilze wie Coniophora puteana und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Herstellungsbeispiele

Darstellung der Zwischenverbindungen

Beispiel 1

350 mmol eines 5-Ring-Lactonderivates VIII und 360 mmol eines Aminderivates V wurden 10 Stunden bei 250°C im Autoklaven unter Eigendruck gerührt und wie üblich aufgearbeitet.

Die Ergebnisse sind in Tabelle 1 zusammengefaßt.

Tabelle 1

Verbindungen X

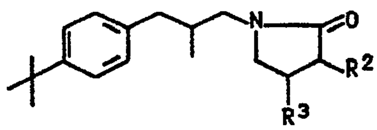

(X)

| Verbindung Nr. | $R^2$ | $R^3$ | Sdp. [°C]/mbar |
|---|---|---|---|
| 1 | n-Hexyl | H | 211-216/0,5 |
| 2 | $-CH_2CH(CH_3)-C_2H_5$ | H | 192-200/0,5 |
| 3 | $-CH_2CH_2-OH$ | H | 220-222/0,2 |

Beispiel 2

Zu 400 mmol eines Derivates des Bernsteinsäureanhydrids VIb in 500 ml Tetrahydrofuran wurden 395 mmol eines Aminderivates V zugetropft. Nach 3 Stunden unter Rückfluß wurde eingeengt und der Rückstand in 1 Liter Essigsäureanhydrid gelöst, nochmals 2 Stunden zum Rückfluß erhitzt und anschließend wie üblich aufgearbeitet.

Die Ergebnisse sind in Tabelle 2 zusammengefaßt.

Tabelle 2

Verbindungen IX

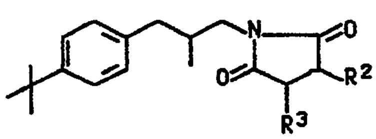

(IX)

| Verbindung Nr. | $R^2$ | $R^3$ | Sdp. [°C]/mbar |
|---|---|---|---|
| 4 | $-CH_3$ | H | 162-163/0,3 |
| 5 | $-CH_2CH=CHCH_3$ | H | 186-188/0,2 |
| 6 | $-(CH_2)_4-$ | | 216-217/0,5 |
| 7 | $-CH_2CH(CH_3)-CH_2CH_2-$ | | 208-210/0,8 |

Darstellung der tert.-Butylphenyl- und tert.-Butylcyclohexyl-isobutylpyrrolidinderivate I

Beispiel 3

Zu einer Suspension von 263 mmol Lithiumaluminiumhydrid in 500 ml Diethylether wurde eine Lösung von 133 mmol eines Succinimidderivates IX in 200 ml Diethylether zugetropft. Nach 4stündigem Rühren bei Raumtemperatur wurde 1 Stunde unter Rückfluß erhitzt und wie üblich aufgearbeitet.

Beispiel 4

Zu 750 ml 99 %iger Essigsäure wurden 1,77 mol eines Aminderivates V, bei 80°C anschließend 1,8 mol 2,5-Dimethoxy-tetrahydrofuranderivates VII zugetropft. Nach einstündigem Rühren bei 80°C wurde wie üblich aufgearbeitet.

353 mmol des Zwischenproduktes wurden in 500 ml Dioxan gelöst und mit $Ru(OH)_3$ bei 130°C und 300 bar hydriert. Nach dem Erreichen der Druckkonstanz ließ man das Reaktionsgemisch erkalten und arbeitete wie üblich auf.

Beispiel 5

Zu 59,4 mmol eines Alkohols, hergestellt in Beispiel 4, in 200 ml Tetrahydrofuran wurden 5,7 g Pyridin und eine Spatelspitze 4-Dimethylaminopyridin (DMAP) zugegeben. Nach Zutropfen von 7,3 g Acetanhydrid und 15-stündigem Rühren bei Raumtemperatur wurde wie üblich aufgearbeitet.

Beispiel 6

Zu 62,7 mmol eines Alkohols, hergestellt in Beispiel 4, in 390 ml Diethylether und 110 ml Dimethylsulfoxid wurde portionsweise 4,2 g Natriumhydrid als 8 %ige Paraffinsuspension zugegeben. Nach 1stündigem Rühren bei Raumtemperatur wurden 126 mmol Methyliodid zugetropft, 1 Stunde zum Rückfluß erhitzt und anschließend wie üblich aufgearbeitet.

Die Ergebnisse der Beispiele 3 bis 6 sind in Tabelle 3 zusammengestellt.

Tabelle 3

Verbindungen I

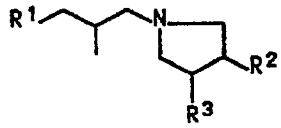

(I)

| Verbindung Nr. | $R^1$ | $R^2$ | $R^3$ | Sdp. [°C]/ mbar |
|---|---|---|---|---|
| 8 | 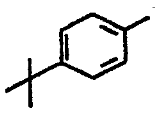 | $-CH_3$ | H | 109-115/0,1 |
| 9 | " | $-CH_2CH(CH_3)CH_2CH_3$ | H | 154-157/0,5 |
| 10 | " | $-n-C_6H_{13}$ | H | 180-184/0,5 |
| 11 | " | $-CH_2CH_2-OH$ | H | 172/0,4 |
| 12 | " | $-CH_2CH_2-OCH_3$ | H | 152-154/0,3 |
| 13 | " | $-CH_2CH_2-OCH_2CH_3$ | H | 165-170/0,6 |
| 14 | " | $-CH_2CH_2-OCH_2CH=CH_2$ | H | 178/0,5 |
| 15 | " | $-CH_2CH_2-O-CO-CH_3$ | H | 170/0,3 |
| 16 | 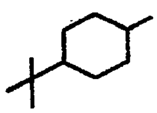 | $-CH_2OH$ | H | 165-168/0,4 |
| 17 | " | $-CH_2-OCH_3$ | H | 160-164/0,8 |
| 18 | " | $-CH_2-OCH_2CH=CH_2$ | H | 152-156/0,3 |
| 19 | " | $-CH_2-O-CO-CH_3$ | H | 188-192/0,4 |
| 20 | " | $—(CH_2)_4—$ | | 165/0,3 |
| 21 | " | $—CH_2CH(CH_3)CH_2CH_2—$ | | 164-167/0,3 |

Darstellung der Salze der tert.-Butylphenyl- und tert.-Butylcyclohexylisobutylpyrrolidinderivate Ia

Beispiel 7

Eine Verbindung I wurde in Ether gelöst. Beim Einleiten von gasförmiger Chlorwasserstoffsäure fiel das entsprechende Hydrochlorid aus, das abgesaugt und im Vakuum getrocknet wurde.

Die Ergebnisse sind in Tabelle 4 zusammengestellt.

Tabelle 4

Verbindungen

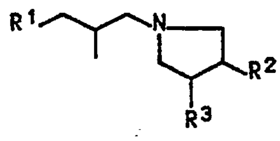 x HCl (Ia)

| Verbindung Nr. | $R^1$ | $R^2$ | $R^3$ | Fp. [°C] |
|---|---|---|---|---|
| 8a | 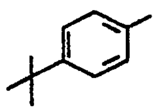 | $-CH_3$ | H | 168-170 |
| 9a | " | $-CH_2CH(CH_3)CH_2CH_3$ | H | 157 |
| 20a | 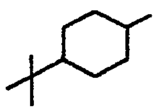 | $-(CH_2)_4-$ | | 194 |
| 22a | 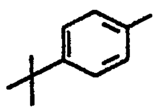 | $-CH_2CH=CHCH_3$ | H | 159 |
| 23a | " | $-CH_2CH(CH_3)CH_2CH_2-$ | | 179 |
| 24a | " | $-(CH_2)_4-$ | | 209 |

Anwendungsbeispiele

Beispiel A

Wirksamkeit gegen Weizenmehltau Sorte

Blätter von in Töpfen gewachsenen Weizenkeimlingen der Sorte Frühgold wurden mit wäßriger Spritzbrühe, die 80 % Wirkstoff und 20 % Emulgiermittel in der Trockensubstanz enthielt, besprüht und 24 Stunden nach dem Antrocknen des Spritzbelages mit Oidien (Sporen des Weizenmehltaus (Erysiphe graminis var. tritici) bestäubt. Die Versuchspflanzen wurden anschließend im Gewächshaus bei Temperaturen zwischen 20 und 22°C und 75 bis 80 % relativer Luftfeuchtigkeit aufgestellt. Nach 7 Tagen wurde das Ausmaß der Mehltauentwicklung ermittelt.

Das Ergebnis des Versuches zeigte, daß die Verbindungen 8 und 11 bis 15 bei der Anwendung als 0,0015 %ige Spritzbrühe eine bessere fungizide Wirkung (mit 90 %) zeigten als die Vergleichsverbindung 3-(4-tert.-Butylphenyl)-2-methylpropylpyrrolidin aus der DE-OS 27 27 482 (mit 60 %). Bei höheren Konzentrationen z.B. als 0,006 %ige Spritzbrühe fand man für die fungizide Wirkung einen Wert von ebenfalls 90 % für die Verbindungen 8 und 11 bis 15 und für die Vergleichsverbindung 70 % Wirksamkeit. Bei der Anwendung einer 0,025 %igen Spritzbrühe erhielt man für die Verbindungen 8 und 11 bis 15 eine fungizide Wirkung von 97 %, für die Vergleichsverbindung 90 %.

Beispiel B

Wirksamkeit gegen Gurkenmehltau

Blätter von in Töpfen gewachsenen Gurkenkeimlingen der Sorte "Chinesische Schlange" wurden im Zweiblattstadium mit einer Sporensuspension des Gurkenmehltaus besprüht. Nach etwa 20 Stunden wurden die Versuchspflanzen mit einer wäßrigen Spritzbrühe, die 80 % Wirkstoff und 20 % Emulgiermittel in

der Trockensubstanz enthielt, bis zur Tropfnässe besprüht. Nach dem Antrocknen des Spritzbelages wurden sie anschließend im Gewächshaus bei Temperaturen zwischen 20 und 22°C und 70 bis 80 % relativer Luftfeuchtigkeit aufgestellt. Zur Beurteilung der Wirksamkeit der neuen Stoffe wurde das Ausmaß der Pilzentwicklung nach 21 Tagen ermittelt.

Das Ergebnis des Versuches zeigte, daß die Verbindungen 8, 9, 10, 12 bis 18 und 21 bei der Anwendung als 0,025 %ige Spritzbrühe eine bessere fungizide Wirkung (mit größer als 97 %) zeigen als die Vergleichsverbindung aus Beispiel A (mit 10 %).

Beispiel C

Wirksamkeit gegen Botrytis cinerea an Paprika

Paprikasämlinge der Sorte "Neusiedler Ideal Elite" wurden, nachdem sich 4-5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22-24°C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedecken.

Das Ergebnis des Versuches zeigte, daß die Verbindungen 12, 13, 15, 16 und 20 bei der Anwendung als 0,05 %ige Spritzbrühe eine bessere fungizide Wirkung (mit 90 %) zeigten als die Vergleichsverbindung aus Beispiel A (mit 60 %).

Beispiel D

Wirksamkeit gegen Plasmopara viticola

Blätter von Topfreben der Sorte "Müller-Thurgau" wurden mit einer wäßrigen Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächsaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus mit Temperaturen zwischen 20 und 30°C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis des Versuches zeigte, daß die Verbindungen 8, 11, 13, 16 und 18 bei der Anwendung als 0,05 %ige Spritzbrühe eine bessere fungizide Wirkung (mit 94 %) zeigen als die Vergleichsverbindung aus Beispiel A (mit 60 %).

**Patentansprüche für die Vertragsstaaten AT, BE, CH, DE, FR, GB, IT, LI, NL, SE**

1. Phenyl- und Cyclohexyl-isobutylpyrrolidinderivate der allgemeinen Formel I

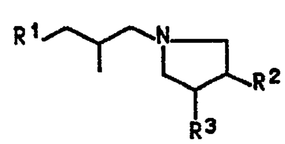

(I)

in der die Substituenten folgende Bedeutung haben:

$R^1$ 4-tert.-Butylphenyl oder 4-tert.-Butylcyclohexyl,

$R^2$ $C_5$–$C_8$-Alkyl, $C_2$–$C_4$-Alkenyl, $C_1$–$C_4$-Alkoxy, $C_2$–$C_4$-Alkenyloxy, $C_1$–$C_4$-Hydroxyalkyl, $C_2$–$C_4$-Alkoxyalkyl, $C_3$–$C_8$-Alkenyloxyalkyl, $C_2$–$C_4$-Acyloxyalkyl, $C_3$–$C_8$-Alkylcarbonylalkyl, und für den Fall, daß $R^1$ 4-tert.-Butylphenyl bedeutet, zusätzlich die Methylgruppe und für den Fall, daß $R^1$ 4-tert.-Butylcyclohexyl bedeutet, zusätzlich $C_1$–$C_4$-Alkyl, die Phenyl-oder Hydroxylgruppe,

$R^3$ Wasserstoff, wobei

$R^2$ und $R^3$ auch gemeinsam einen carbocyclischen Rest bedeuten können,

sowie deren pflanzenphysiologisch verträgliche Salze.

2. Verfahren zur Herstellung der Phenyl-und Cyclohexyl-isobutylpyrrolidinderivate I, dadurch gekennzeichnet, daß man

a) eine Verbindung II,

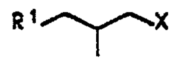

(II)

in der der Substituent X einen in basischen Milieu abspaltbaren Rest bedeutet, unter basischen Bedingungen mit einem Pyrrolidinderivat III

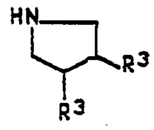

(III)

umsetzt oder

b) einen Aldehyd der Formel IV

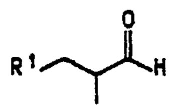

(IV)

mit einer Verbindung III

$b_1$) entweder unter reduzierenden Bedingungen oder

$b_2$) das entstehende Enamin in an sich bekannter Weise durch katalytische Hydrierung umsetzt oder

c) ein Aminderivat der Formel V

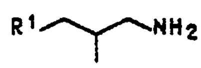

(V)

$c_1$) mit einer Dicarbonsäure, einem Dicarbonsäurediester oder Dicarbonsäuredihalogenid alle der allgemeinen Formel

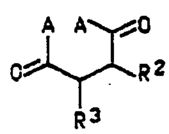

(VIa)

in der der Rest A Hydroxy, Alkoxy oder Halogen bedeutet, oder

$c_2$) mit einer Verbindung VIb

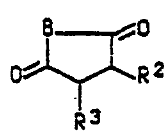

(VIb)

in der B für Sauerstoff oder NH steht, oder

$c_3$) mit einem Lacton bzw. Lactam der allgemeinen Formel VIc

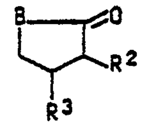

(VIc)

in an sich bekannter Weise umsetzt, wobei $R^1$, $R^2$ und $R^3$ die in Anspruch 1 genannten Bedeutungen haben, und die Carbonylgruppe(n) ebenfalls in an sich bekannter Weise zu I reduziert und anschließend gegebenenfalls in an sich bekannter Weise in deren Salze überführt.

3. Verfahren zur Herstellung der Verbindungen Ia

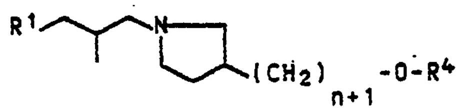

(Ia)

$R^4$ = H, $C_1$–$C_4$-Alkyl, $C_3$–$C_8$-Alkylcarbonyl, $C_2$–$C_4$-Alkenyl, $C_2$–$C_4$-Acyl
n = 0 bis 3
wobei $R^1$ die in Anspruch 1 genannten Bedeutungen hat, dadurch gekennzeichnet, daß man 2,5-Dimethoxy-tetrahydrofuranderivate VII

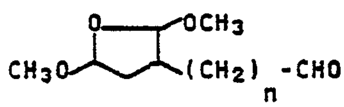

(VII)

mit Aminderivaten der Formel V gemäß Anspruch 2 unter Entfernung des Reaktionswassers umsetzt und das entstandene Pyrrolaldehydderivat mit Wasserstoff in Gegenwart eines Edelmetallkatalysators hydriert und gegebenenfalls mit einer Verbindung $R^4$–X, in der X eine Abgangsgruppe bedeutet, verethert bzw. verestert.

4. Verfahren zur Herstellung der 4-tert.-Butylcyclohexyl-isobutylpyrrolidinderivate der Formel I, dadurch gekennzeichnet, daß man die Verbindungen I, in denen $R^1$ = 4-tert.-Butylphenyl bedeutet mit Wasserstoff in Gegenwart eines Edelmetallkatalysators in an sich bekannter Weise umsetzt.

5. Verwendung von Phenyl-und Cyclohexyl-isobutylpyrrolidinderivaten I gemäss Anspruch 1 als Fungizide.

6. Fungizid, enthaltend eine Verbindung der Formel I gemäß Anspruch 1 neben hierfür üblichen Trägerstoffen.

7. Verfahren zur Bekämpfung von Pilzen, dadurch gekennzeichnet, daß man die Pilze oder die von Pilzbefall bedrohten Materialien, Pflanzen, Saatgüter oder Böden mit einer Verbindung der Formel I gemäß Anspruch 1 behandelt.

**Patentansprüche für die Vertragsstaaten: ES, GR**

1. Verfahren zur Herstellung der Phenyl- und Cyclohexyl-isobutylpyrrolidinderivate der allgemeinen Formel I

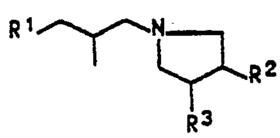

(I)

in der die Substituenten folgende Bedeutung haben:
$R^1$ 4-tert.-Butylphenyl oder 4-tert.-Butylcyclohexyl,
$R^2$ $C_5$–$C_8$-Alkyl, $C_2$–$C_4$-Alkenyl, $C_1$–$C_4$-Alkoxy, $C_2$–$C_4$-Alkenyloxy, $C_1$–$C_4$-Hydroxyalkyl, $C_2$–$C_4$-Alkoxyalkyl, $C_3$–$C_8$-Alkenyloxyalkyl, $C_2$–$C_4$-Acyloxyalkyl, $C_3$–$C_8$-Alkylcarbonylalkyl, und für den Fall, daß $R^1$ 4-tert.-Butylphenyl bedeutet, zusätzlich die Methylgruppe, und für den Fall, daß $R^1$ 4-tert.-Butylcyclohexyl bedeutet, zusätzlich $C_1$–$C_4$-Alkyl, die Phenyl-oder Hydroxylgruppe,
$R^3$ Wasserstoff, wobei
$R^2$ und $R^3$ auch gemeinsam einen carbocyclischen Rest bedeuten können, sowie deren pflanzenphysiologisch verträgliche Salze, dadurch gekennzeichnet, daß man

a) eine Verbindung II,

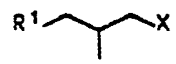

(II)

in der der Substituent X einen in basischen Milieu abspaltbaren Rest bedeutet, unter basischen Bedingungen mit einem Pyrrolidinderivat III

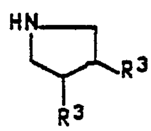

(III)

umsetzt oder

b) einen Aldehyd der Formel IV

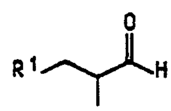

(IV)

mit einer Verbindung III

$b_1$) entweder unter reduzierenden Bedingungen oder

$b_2$) das entstehende Enamin in an sich bekannter Weise durch katalytische Hydrierung umsetzt oder

c) ein Aminderivat der Formel V

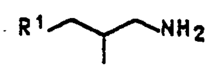

(V)

$c_1$) mit einer Dicarbonsäure, einem Dicarbonsäurediester oder Dicarbonsäuredihalogenid alle der allgemeinen Formel VIa

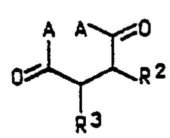

(VIa)

in der der Rest A Hydroxy, Alkoxy oder Halogen bedeutet, oder

$c_2$) mit einer Verbindung VIb

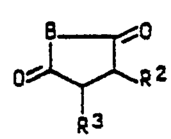

(VIb)

in der B für Sauerstoff oder NH steht, oder

$c_3$) mit einem Lacton bzw. Lactam der allgemeinen Formel VIc

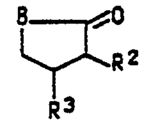

(VIc)

in an sich bekannter Weise umsetzt und die Carbonylgruppe(n) ebenfalls in an sich bekannter Weise zu I reduziert, und anschließend gegebenenfalls in an sich bekannter Weise in deren Salze überführt.

2. Verfahren zur Herstellung der Verbindungen Ia

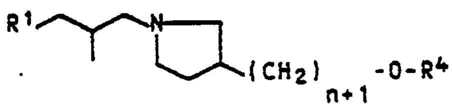

(Ia)

$R^4$ =H, $C_1$–$C_4$-Alkyl, $C_3$–$C_8$-Alkylcarbonyl, $C_2$–$C_4$-Alkenyl, $C_2$–$C_4$-Acyl
n = 0 bis 3
wobei $R^1$ die in Anspruch 1 genannte Bedeutung hat, dadurch gekennzeichnet, daß man 2,5-Dimethoxytetrahydrofuranderivate VII

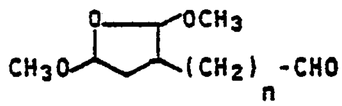

(VII)

mit Aminderivaten der Formel V gemäß Anspruch 1 unter Entfernung des Reaktionswassers umsetzt und das entstandene Pyrrolaldehydderivat mit Wasserstoff in Gegenwart eines Edelmetallkatalysators hydriert und gegebenenfalls mit einer Verbindung $R^4$–X, in der X eine Abgangsgruppe bedeutet, verethert bzw. verestert.

3. Verfahren zur Herstellung der 4-tert.-Butylcyclohexyl-isobutylpyrrolidinderivate der Formel I, dadurch gekennzeichnet daß man die Verbindungen I, in denen $R^1$ = 4-tert.-Butylphenyl bedeutet mit Wasserstoff in Gegenwart eines Edelmetallkatalysators in an sich bekannter Weise umsetzt.

4. Verwendung von Phenyl-und Cyclohexyl-isobutylpyrrolidinderivaten I gemäß Anspruch 1 als Fungizide.

5. Fungizid, enthaltend eine Verbindung der Formel I gemäß Anspruch 1 neben hierfür üblichen Trägerstoffen.

6. Verfahren zur Bekämpfung von Pilzen, dadurch gekennzeichnet, daß man die Pilze oder die von Pilzbefall bedrohten Materialien, Pflanzen, Saatgüter oder Böden mit einer Verbindung der Formel I gemäß Anspruch 1 behandelt.

**Claims for the Contracting States AT, BE, CH, DE, FR, GB, IT, LI, NL, SE**

1. Phenyl- or cyclohexylisobutylpyrrolidine derivative of the formula I

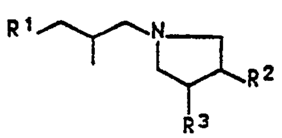

(I)

where
$R^1$ is 4-tert-butylphenyl or 4-tert-butylcyclohexyl,
$R^2$ is $C_5$–$C_8$-alkyl, $C_2$–$C_4$-alkenyl, $C_1$–$C_4$-alkoxy, $C_2$–$C_4$-alkenyloxy, $C_1$–$C_4$-hydroxyalkyl, $C_2$–$C_4$-alkoxyalkyl, $C_3$–$C_8$-alkenyloxyalkyl, $C_2$–$C_4$-acyloxyalkyl or $C_3$–$C_8$-alkylcarbonylalkyl or, where
$R^1$ is 4-tert-butylphenyl, may furthermore be methyl and, where $R^1$ is 4-tert-butylcyclohexyl, may furthermore be $C_1$–$C_4$-alkyl, phenyl or hydroxyl,

$R^3$ is hydrogen, and
$R^2$ and $R^3$ together may furthermore be a carbocyclic radical, and its phytophysiologically tolerated salts.

2. A process for the preparation of phenyl- or cyclohexylisobutylpyrrolidine derivatives I wherein
a) a compound II

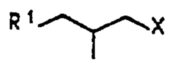

(II)

where X is a radical which can be eliminated in a basic medium, is reacted with pyrrolidine derivative III

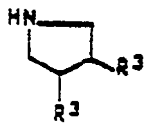

(III)

under basic conditions, or
b) an aldehyde of the formula IV

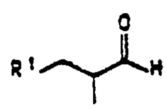

(IV)

is reacted with a compound
$b_1$) either under reducing conditions or
$b_2$) the resulting enamine is converted in a conventional manner by catalytic hydrogenation, or
c) an amine derivative of the formula V

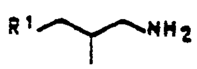

(V)

is reacted
$c_1$) with a dicarboxylic acid, a dicarboxylic diester or a diacyl dihalide, all of the formula VIa

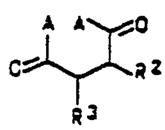

(VIa)

where A is hydroxyl, alkoxy or halogen, or
$c_2$) with a compound VIb

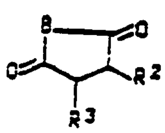

(VIb)

where B is oxygen or NH, or
$c_3$) with a lactone or lactam of the formula VIc

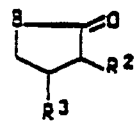

(VIc)

in a conventional manner, $R^1$, $R^2$ and $R^3$ having the meanings stated in claim 1 and the carbonyl group or groups is or are reduced, likewise in a conventional manner, to give I, and, if required, the product is then converted into its salts in a conventional manner.

3. A process for the preparation of a compound Ia,

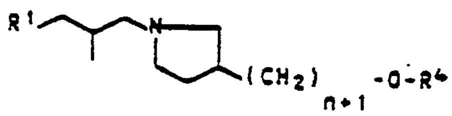

(Ia)

where $R^1$ has the meanings stated in claim 1, $R^4$ is H, $C_1$–$C_4$-alkyl, $C_3$–$C_8$-alkylcarbonyl, $C_2$–$C_4$-alkenyl or $C_2$–$C_4$-acyl and n is 0 to 3, wherein a 2,5-dimethoxytetrahydrofuran derivative VII

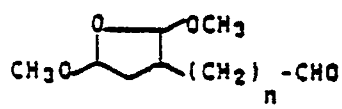

(VII)

is reacted with an amine derivative of the formula V as claimed in claim 1 with removal of the water of reaction, and the resulting pyrrolecarbaldehyde derivative is hydrogenated with hydrogen in the presence of a noble metal catalyst and, if required, etherified or esterified with a compound $R^4$–X, where X is a leaving group.

4. A process for the preparation of a 4-tert-butylcyclohexylisobutylpyrrolidine derivative of the formula I, wherein a compound I, where $R^1$ is 4-tert-butylphenyl, is reacted with hydrogen in the presence of a noble metal catalyst in a conventional manner.

5. Use of a phenyl- or cyclohexylisobutylpyrrolidine derivative I as claimed in claim 1 as a fungicide.

6. A fungicide containing a compound of the formula I as claimed in claim 1 in addition to conventional carriers for this purpose.

7. A method for controlling fungi, wherein the fungi or the materials, plants, seeds or soils threatened by fungal attack are treated with a compound of the formula I as claimed in claim 1.

**Claims for the Contracting States: ES, GR**

1. A process for the preparation of a phenyl- or cyclohexylisobutylpyrrolidine derivative of the formula I

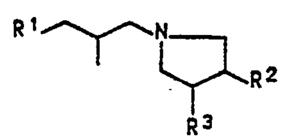

(I)

where
$R^1$ is 4-tert-butylphenyl or 4-tert-butylcyclohexyl,
$R^2$ is $C_5$–$C_8$-alkyl, $C_2$–$C_4$-alkenyl, $C_1$–$C_4$-alkoxy, $C_2$–$C_4$-alkenyloxy, $C_1$–$C_4$-hydroxyalkyl, $C_2$–$C_4$-alkoxyalkyl, $C_3$–$C_8$-alkenyloxyalkyl, $C_2$–$C_4$-acyloxyalkyl or $C_3$–$C_8$-alkylcarbonylalkyl or, where $R^1$ is 4-tert-butylphenyl, may furthermore be methyl and, where $R^1$ is 4-tert-butylcyclohexyl, may furthermore be $C_1$–$C_4$-alkyl, phenyl or hydroxyl,
$R^3$ is hydrogen, and
$R^2$ and $R^3$ together may furthermore be a carbocyclic radical, and its phytophysiologically tolerated salts, wherein
a) a compound II

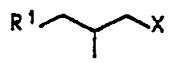

(II)

where X is a radical which can be eliminated in a basic medium, is reacted with a pyrrolidine derivative III

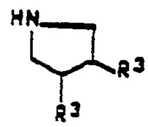

(III)

under basic conditions, or

b) an aldehyde of the formula IV

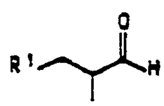

(IV)

is reacted with a compound III

$b_1$) either under reducing conditions or

$b_2$) the resulting enamine is converted in a conventional manner by catalytic hydrogenation, or

c) an amine derivative of the formula V

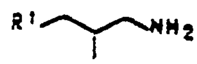

(V)

is reacted

$c_1$) with a dicarboxylic acid, a dicarboxylic diester or a diacyl dihalide, all of the formula VIa

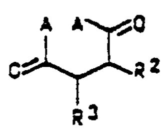

(VIa)

where A is hydroxyl, alkoxy or halogen, or

$c_2$) with a compound VIb

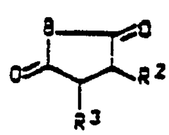

(VIb)

where B is oxygen or NH, or

$c_3$) with a lactone or lactam of the formula VIc

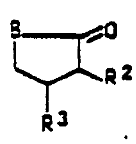

(VIc)

in a conventional manner, and the carbonyl group or groups is or are reduced, likewise in a conventional manner, to give I, and, if required, the product is then converted into its salts in a conventional manner.

2. A process for the preparation of a compound Ia,

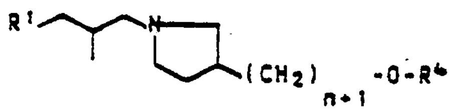

(Ia)

where $R^1$ has the meanings stated in claim 1, $R^4$ is H, $C_1$–$C_4$-alkyl, $C_3$–$C_8$-alkylcarbonyl, $C_2$–$C_4$-alkenyl or $C_2$–$C_4$-acyl and n is 0 to 3, wherein a 2,5-dimethoxytetrahydrofuran derivative VII

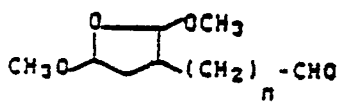

(VII)

is reacted with an amine derivative of the formula V as claimed in claim 1 with removal of the water of reaction, and the resulting pyrrolecarbaldehyde derivative is hydrogenated with hydrogen in the presence of a noble metal catalyst and, if required, etherified or esterified with a compound $R^4$–X, where X is a leaving group.

3. A process for the preparation of a 4-tert-butylcyclohexylisobutylpyrrolidine derivative of the formula I, wherein a compound I, where $R^1$ is 4-tert-butylphenyl, is reacted with hydrogen in the presence of a noble metal catalyst in a conventional manner.

4. Use of a phenyl- or cyclohexylisobutylpyrrolidine derivative I as claimed in claim 1 as a fungicide.

5. A fungicide containing a compound of the formula I as claimed in claim 1 in addition to conventional carriers for this purpose.

6. A method for controlling fungi, wherein the fungi or the materials, plants, seeds or soils threatened by fungal attack are treated with a compound of the formula I as claimed in claim 1.

**Revendications pour les Etats contractants: AT, BE, CH, DE, FR, GB, IT, LI, NL, SE**

1. Dérivés de phényl- et cyclohexyl-isobutylpyrrolidine de formule générale

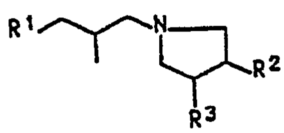

(I)

dans laquelle les substituants ont les significations suivantes:

$R^1$, 4-tert-butyl-phényle ou 4-tert-butylcyclohexyle

$R^2$, alkyle en $C_5$–$C_8$, alcényle en $C_2$–$C_4$, alcoxy en $C_1$–$C_4$, alcényloxy en $C_2$–$C_4$, hydroxyalkyle en $C_1$–$C_4$, alcoxyalkyle en $C_2$–$C_4$, alcényloxyalkyle en $C_3$–$C_8$, acyloxyalkyle en $C_2$–$C_4$, alkylcarbonylalkyle en $C_3$–$C_8$, et dans le cas où $R^1$ signifie 4-tert-butylphényle, additionellement le groupe méthyle et, dans le cas où $R^1$ représente 4-tert-butylcyclohexyle, additionellement alkyle en $C_1$–$C_4$, le groupe phényle ou hydroxyle

$R^3$, hydrogène

$R^2$ et $R^3$ pouvant aussi signifier, ensemble, un reste carbocyclique

ainsi que leurs sels acceptables physiologiquement par les plantes.

2. Procédé pour la préparation des dérivés de phényl- et cyclohexyl-isobutylpyrrolidine, caractérisé par le fait que:

a) on fait réagir un composé II

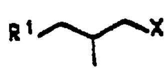

(II)

dans lequel le substituant X représente un reste séparable en milieu basique, sous conditions basiques, avec un dérivé de pyrrolidine III

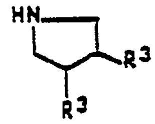

(III)

ou

b) on fait réagir un aldéhyde de formule IV

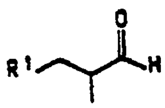

(IV)

avec un composé III

$b_1$) soit sous conditions réductrices, soit

$b_2$) en utilisant l'enamine obtenue, de manière connue en soi, par hydrogénation catalytique

ou

c) on fait réagir, de manière connue en soi, un dérivé amine de formule V

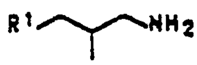

(V)

$c_1$) avec un acide dicarboxylique, un diester dicarboxylique ou un dihalogénure d'acide dicarboxylique, tous de la formule générale VIa

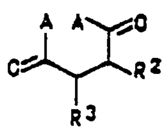

(VIa)

dans laquelle le reste A représente hydroxy, alcoxy ou halogène, ou

$c_2$) avec un composé VIb

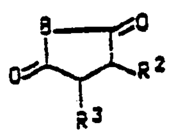

(VIb)

dans lequel B est mis pour oxygène ou NH, ou

$c_3$)avec une lactone ou un lactame de formule générale VIc

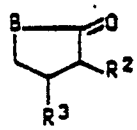

(VIc)

$R^1$, $R^2$ et $R^3$ ayant les significations indiquées dans la revendication 1, et on réduit, également de manière connue en soi et on transforme éventuellement, de manière connue en soi, en leurs sels.

3. Procédé de préparation des composés Ia

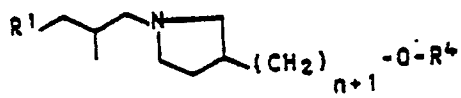

(Ia)

$R^1$ ayant les significations données dans la revendication 1
$R^4$ = H, alkyle en $C_1$–$C_4$, alkylcarbonyle en $C_3$–$C_8$, alcényle en $C_2$–$C_4$ et acyle en $C_2$–$C_4$, n = 0 à 3 caractérisé par le fait que l'on fait réagir des dérivés de 2,5-diméthoxy-tétrahydrofuranne VII

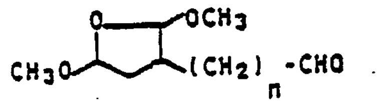

(VII)

avec des dérivés aminés de formule V selon la revendication 2 en éliminant l'eau de réaction, et on hydrogène le dérivé de pyrrole-aldéhyde obtenu, avec de l'hydrogène, en présence d'un catalyseur en métal noble et éventuellement on éthérifie ou estérifie avec un composé $R^4$–X, où X représente un groupe séparable.

4. Procédé de préparation de dérivés de 4-tert-butylcyclohexyl-isobutylpyrrolidine de formule I, caractérisé par le fait que l'on fait réagir, de manière connue en soi, les composés I, dans lesquels $R^1$ représente 4-tert-butylphényle avec de l'eau en présence d'un catalyseur en métal noble.

5. Utilisation des dérivés de phényl- et cyclohexylisobutylpyrrolidine selon la revendication 1 comme fongicides.

6. Fongicide contenant un composé de formule I selon la revendication 1, outre des matières supports usuelles pour cela.

7. Procédé de lutte contre les champignons, caractérisé par le fait que l'on traite les champignons ou les materiaux, plantes, semences ou sols menacés d'une attaque par les champignons, avec un composé de formule I, selon la revendication 1.

**Revendications pour les Etats contractants: ES, GR**

1. Procédé pour la préparation de dérivés de phényl- et cyclohexyl-isobutylpyrrolidine de formule générale

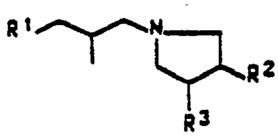

(I)

dans laquelle des substituants ont les significations suivantes:
$R^1$, 4-tert-butyl-phényle ou 4-tert-butylcyclohexyle
$R^2$, alkyle en $C_5$–$C_8$, alcényle en $C_2$–$C_4$, alcoxy en $C_1$–$C_4$, alcényloxy en $C_2$–$C_4$, hydroxyalkyle en $C_1$–$C_4$, alcoxyalkyle en $C_2$–$C_4$, alcényloxyalkyle en $C_3$–$C_8$, acyloxyalkyle en $C_2$–$C_4$, alkylcarbonylalkyle en $C_3$–$C_8$, et dans le cas où $R^1$ signifie 4-tert-butylphényle, additionellement le groupe méthyle et, dans le cas où $R^1$ représente 4-tert-butylcyclohexyle, additionellement alkyle en $C_1$–$C_4$, le groupe phényle ou hydroxyle
$R^3$, hydrogène
$R^2$ et $R^3$ pouvant aussi signifier, ensemble, un reste carbocyclique
ainsi que leurs sels acceptables physiologiquement par les plantes, caractérisé par le fait que:

a) on fait réagir un composé II

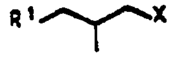

(II)

dans lequel le substituant X représente un reste séparable en milieu basique, sous conditions basiques, avec un dérivé de pyrrolidine III

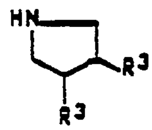

(III)

ou

b) on fait réagir un aldéhyde de formule IV

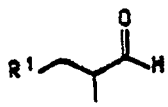

(IV)

avec un composé III

$b_1$) soit sous conditions réductrices, soit

$b_2$) en utilisant l'enamine obtenue, de manière connue en soi, par hydrogénation catalytique

ou

c) on fait réagir, de manière connue en soi, un dérivé amine de formule V

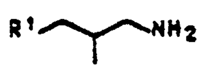

(V)

$c_1$) avec un acide dicarboxylique, un diester dicarboxylique ou un dihalogénure d'acide dicarboxylique, tous de la formule générale VIa

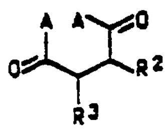

(VIa)

dans laquelle le reste A représente hydroxy, alcoxy ou halogène, ou

$c_2$) avec un composé VIb

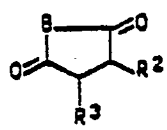

(VIb)

dans lequel B est mis pour oxygène ou NH, ou

$c_3$) avec une lactone ou un lactame de formule générale VIc

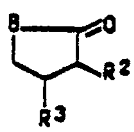

(VIc)

$R^1$, $R^2$ et $R^3$ ayant les significations indiquées dans la revendication, et on réduit, également de manière connue en soi et on transforme éventuellement, de manière connue en soi, en leurs sels.

2. Procédé de préparation des composés Ia

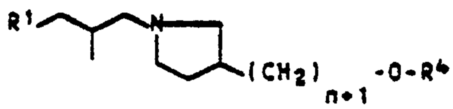

(Ia)

$R^1$ ayant les significations données dans la revendication 1
$R^4$ = H, alkyle en $C_1$–$C_4$, alkylcarbonyle en $C_3$–$C_8$, alcényle en $C_2$–$C_4$ et acyle en $C_2$–$C_4$, n = 0 à 3, caractérisé par le fait que l'on fait réagir des dérivés de 2,5-dimethoxy-tétrahydrofuranne VII

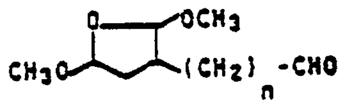

(VII)

avec des dérivés aminés de formule V selon la revendication 2 en éliminant l'eau de réaction, et on hydrogène le dérivé de pyrrole-aldéhyde obtenu, avec de l'hydrogène, en présence d'un catalyseur en métal noble et éventuellement on éthérifie ou estérifie avec un composé $R^4$–X, où X représente un groupe séparable.

3. Procédé de préparation de dérivés de 4-tert-butylcyclohexyl-isobutylpyrrolidine de formule I, caractérisé par le fait que l'on fait réagir, de manière connue en soi, les composés I, dans lesquels $R^1$ représente 4-tert-butylphényle avec de l'eau en présence d'un catalyseur en métal noble.

4. Utilisation des dérivés de phényl- et cyclohexyl-isobutylpyrrolidine selon la revendication 1 comme fongicides.

5. Fongicide contenant un composé de formule I selon la revendication 1, outre des matières supports usuelles pour cela.

6. Procédé de lutte contre les champignons, caractérisé par le fait que l'on traite les champignons ou les matériaux, plantes, semences ou sols menacés d'une attaque par les champignons, avec un composé de formule I, selon la revendication 1.